# EUROPÄISCHE PATENTANMELDUNG

(11) **EP 1 857 080 A2**
(43) Veröffentlichungstag der Anmeldung: **21.11.2007**
(21) Anmeldenummer: 07008534.5
(22) Anmeldetag: 26.04.2007
(51) Int. Cl.: A61F 2/52, B29C 35/00

(54) **Verfahren zur Herstellung einer Brustprothese**

(30) Priorität: 19.05.2006 DE 102006023675; 28.07.2006 DE 102006035069
(71) Anmelder: AMOENA Medizin-Orthopädie-Technik GmbH, 83064 Raubling (DE)
(72) Erfinder: Reitmeier, Paul, 83547 Babenshan (DE); Wild, Helmut, 83022 Rosenheim (DE)
(74) Vertreter: Laufhütte, Dieter

(57) **Zusammenfassung**

Verfahren zur Herstellung einer Brustprothese, bei dem ein Folienbeutel aus mindestens drei Folienlagen zur Erzeugung von mindestens zwei Kammern verschweißt wird.

## Beschreibung

Die Erfindung betrifft ein Verfahren zur Herstellung einer Brustprothese, die im Wesentlichen aus in Kunststofffolien eingeschweißten, der Brustform nachgebildeten Körpern besteht.

Es sind bereits Brustprothesen bekannt, die zur Gewichtsreduzierung mit Leichtsilikon befüllt sind. Derartiges Leichtsilikon besteht aus einem Standardsilikon mit Beimischung von Hohlkugelfüllstoffen zur Gewichtseinsparung. Diese Brustprothesen gewinnen kontinuierlich an Marktanteil. Der überwiegende Anteil von Brustprothesen wird in Spezialbüstenhalter oder anderen besonders geeigneten, mit Taschen ausgestatteten Büstenhaltern getragen. Das gesamte Gewicht der Prothesen muß deshalb vom Büstenhalter und somit hauptsächlich von Büstenhalterträger aufgenommen werden. Diese Gewichtsbelastung wird besonders bei großen Größen als unangenehm empfunden. Leichtprothesen mit einer Gewichtseinsparung von 25 bis 35 Gew.% erhöhen daher deutlich den Tragekomfort und zeigen darüber hinaus noch genügend natürliches Bewegungsverhalten.

Das vornehmlich bei Brustprothesen eingesetzte Silikonmaterial ist von Natur aus transparent. Die gewünschte Hautfarbe kann problemlos beispielsweise durch Zugabe von geringen Anteilen von Farbpigmenten - beispielsweise einer Zumischung von 0,2 Gew.% Farbpigment - erreicht werden. Typischerweise werden als das Silikon umschließende Folien Polyurethanfolien eingesetzt. Diese weisen eine hohe Elastizität und Weichheit auf. Dennoch sind sie im Vergleich zu dem verwendeten Silikon eher steif, so dass die Folie zur Faltenbildung neigt. Aufgrund der Lichtdurchlässigkeit der Polyurethanfolie und des Silikons sind diese Falten aber nicht deutlich sichtbar und stören deshalb nicht.

Durch Hinzugabe von Hohlkugelfüllstoffen, die wiederum aus lichtdurchlässigem Kunststoff bestehen, ergibt sich eine stark deckende weiße Farbe in dem Leichtsilikon. Dies rührt daher, dass die Hohlkugelfüllstoffe aus einer Kugelvielzahl bestehen, die einzeln als Farbprisma wirken. Diese Erscheinung ist beispielsweise mit transparenten Schneekristallen vergleichbar, die aufgrund der Lichtbrechung in ihrer Summe ebenfalls weiß aussehen. Die weiße Farbe des Leichtsilikons kann durch die Zugabe von Farbpigmenten in Richtung einer Angleichung an die Hautfarbe verändert werden. Diese wird aber nicht so natürlich wiedergegeben, wie dies bei Standardsilikon möglich ist. Aufgrund dieser farblichen Unterschiede werden die zuvor erwähnten Falten der Polyurethanfolie durch die deckende Farbe des Leichtsilikons wesentlich stärker sichtbar und werden optisch als störend empfunden.

Zur Vermeidung des vorgenannten Problems ist es bereits bekannt geworden, eine mehrschichtige Brustprothese zu schaffen, wobei außen eine dünne Deckschicht aus Standardsilikon auf der Vorderseite gebildet wird, welches transparent ist und mit der an die Hautfarbe angepassten Farbgebung ein attraktives Erscheinungsbild ergibt.

Aus der EP 0 880 951 B1 ist ein Verfahren zur Herstellung einer Mehrkammerbrustprothese bekannt, bei dem die Herstellung der Kosmetikschicht außerhalb der Prothesenform erfolgt.

Aufgabe der vorliegenden Erfindung ist es nun, ein Verfahren zu entwickeln, dass eine derartige Prothese mit einer sogenannten kosmetischen Deckschicht in einfacher Art und Weise zu schaffen.

Erfindungsgemäß wird diese Aufgabe durch ein Verfahren nach dem Anspruch 1 geschaffen.

Das erfindungsgemäße Verfahren zur Herstellung der Brustprothese, die im wesentlichen ja aus in Kunststofffolien eingeschweißten, dem Brustraum nachgebildeten Körpern besteht, besteht aus folgenden Schritten:
- Schweißen eines Folienbeutels aus mindestens drei Folienlagen zur Erzeugung von mindestens zwei Kammern,
- Einlegen des Folienbeutels in das Prothesenformwerkzeug - Befüllen der außen liegenden Kammer mit einer vorzugsweise bereits bei Umgebungstemperatur oder bei leicht erhöhter Temperatur vernetzenden, transparenten Masse,
- Teilweises oder vollständiges Aushärten der transparente Masse, um eine ausreichende Formstabilität für die weiteren Fertigungsschritte zu erreichen
- Befüllen der inneren Kammer mit einer zweiten, weitgehend lichtundurchlässigen Masse, gefüllt mit Leichtfüllstoffen
- Einsetzen einer Formhälfte mit einer flachen, die Endkontur definierende Höhlung (=rückseitge Formenhälfte), um bei erhöhter Temperatur die in den Kammern befindliche Massen vollständig zu vernetzen.
- Die optionale dritte Kammer kann entweder zusammen mit der äußeren Schicht gefüllt werden, in einer Schichtdicke von vorzugsweise 2-10 mm, in der gleichen Funktion wie die äußere Schicht oder
   sie wird zusammen der inneren Kammer gefüllt, in einer Weichheit und Menge wie in dem Amoena Patent EP 0 320 590 (Mulligan) beschrieben.

Dadurch dass die äußere dünne Schicht bereits bei Umgebungstemperatur oder bei nur leicht erhöhter Temperatur vernetzt, kann die Zeit zur Herstellung der gesamten Brustprothese erheblich verringert werden. Insgesamt ergibt sich ein einfaches Verfahren, das zur Herstellung von optisch einwandfreien Brustprothesen in hoher Qualität führt.

Besondere Vorteile der Erfindung ergeben sich aus den sich an den Hauptanspruch anschließenden Unteransprüchen.

Demnach kann vorteilhaft zur Bildung der dünnen äußeren Schicht in der äußeren Kammer des Folienbeutels eine Formhälfte mit einer Tiefenhöhlung auf die erste Formhälfte aufgesetzt werden, so dass durch den Spalt zwischen den Formhälften die Schichtdicke des äußeren Schicht festgelegt wird.

Alternativ zu dieser Ausbildung der äußeren Schicht, der sogenannten kosmetischen Schicht, kann statt des Einsetzens eine Formhälfte mit tiefer Höhlung eine dünnere äußere Schicht in der äußeren Kammer auch durch entsprechenden Druck und/oder Vakuum, der auf den in der Form eingespannten aus elastischem Material bestehenden Folienbeutel ausgeübt wird, hervorgerufen werden, so dass sich die Kontur der gewünschten Höhlung ergibt. Aufgrund dieser Verfahrensweise ist bei der Erstellung der Form keine so hohe Präzision erforderlich. Bei dieser Verfahrensvariante wird vorteilhaft auch der Folienbeutel bei der Vernetzung der äußeren Schicht nicht so stark gedehnt.

Vorteilhaft bestehen die den Folienbeutel bildenden Folienlagen aus Polyurethan. Die Polyurethanfolie weist dabei vorteilhaft ein Dicke von 40 µm bis 100 µm auf. Die Polyurethanfolien können vorteilhaft mittels Thermoschweißen ,HF-Schweißen oder Laserschweißen miteinander verschweißt werden.

Die in der ersten Kammer des Folienbeutels eingefüllte erste Masse, die die kosmetische Außenschicht bildet, besteht aus einer Silikonmischung, die so eingestellt ist, dass vorteilshaft schon bei Raumtemperatur hinreichend stark vernetzt, um nach wenigen Minuten Reaktionszeit weitgehend formstabil zu sein. Es kann auch eine Silikonmischung gewählt werden, die diese Eigenschaften bei vergleichsweise geringeren Temperaturen in kurzer Zeit erreicht.

Vorteilhaft ist die in der ersten Kammer des Folienbeutels eingefüllten Masse Farbpigmente beigemengt.

Die Weichheit des in der ersten Kammer des Folienbeutels eingeführten Silikons beträgt nach dem Vernetzen in Penetrationseinheiten gemessen zwischen 170 und 230 Einheiten.

Zur vollständigen Vernetzung der in der zweiten Kammer des Folienbeutels eingefüllten Masse wird die Temperatur vorteilhaft so gewählt, dass das Folienmaterial darauf plastisch verformt wird, ohne dabei die Folie dauerhaft zu beschädigen. Dabei kann bei Verwendung von Polyurethan als Folienmaterial die Temperatur auf ca. 130 °C eingestellt werden, um hinreichend schnell eine gute Vernetzung des Silikons und eine entsprechend Verformung der Polyurethanfolie zu erreichen.

Gemäß einer weiteren vorteilhaften Ausgestaltung der Erfindung kann der Folienbeutel aus vier Folienlagen unter Bildung von drei Kammern gefertigt sein, wobei die vergleichsweise dünne dritte Kammer auf der Rückseite der Folie angeordnet ist. Auch diese vergleichsweise dünne Kammer stellt eine sogenannte kosmetische Schicht dar.

Die erste Kammer kann eine Dicke von ca. 2 bis 10 mm aufweisen. Die dritte Kammer kann entweder ähnlich der ersten Kammer als Kosmetikschicht in vergleichbarer Dicke gefertigt sein oder entsprechend der weichen Schicht wie in EP 0 320 590 (Mulligan) beschrieben.

Weitere Merkmale, Einzelheiten und Vorteile der Erfindung werden anhand eines in der Zeichnung dargestellten Ausführungsbeispiels erläutert. Es zeigen:
- Fig. 1:: eine schematische Darstellung einer Form zur Bildung einer Brustprothese mit einem eingelegten Folienbeutel, in dem nur die äußere Schicht mit Silikon befüllt ist, gemäß einem Zwischenschritt des erfindungsgemäßen Verfahrens,
- Fig. 2:: eine Darstellung gemäß Fig. 1, in welcher auch die zweite Kammer befüllt ist und
- Fig. 3:: eine schematische Darstellung einer Form zur Bildung einer Brustprothese mit einem eingelegten Folienbeutel, in dem nur die äußere Schicht mit Silikon befüllt ist, gemäß einem Zwischenschritt mit membranähnlich ausgeformten Höhlung des erfindungsgemäßen Verfahrens, .

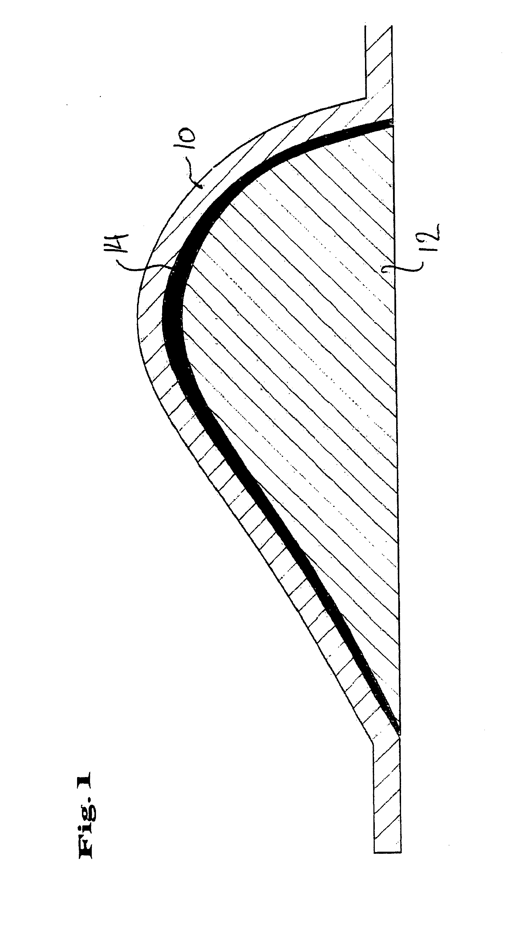

Bei der Herstellung der erfindungsgemäßen Brustprothese wird zunächst ein Folienbeutel aus mindestens drei Folienlagen geschweißt. Soweit drei Folienlagen verwendet werden, werden zwei Kammern gebildet. Alternativ können aber auch vier Folienlagen miteinander verschweißt werden, so dass drei Kammern entstehen. Die verwendete Folie besteht aus Polyurethan in einer Stärke von typischerweise 40 µm bis 100 µm. Die Verschweißung der Folienlagen erfolgt durch Thermoschweißen , HF-Schweißen oder Laserschweißen. Dieser Folienbeutel wird in die Prothesenform eingelegt. In Fig. 1 ist die Prothesenform schematisch dargestellt. Sie besteht im wesentlichen aus zwei Formhälften 10 und 12. Der genaue Aufbau der Form muß an dieser Stelle nicht erläutert werden, da er zum Stand der Technik gehört.

Die Folien sind anfänglich nicht vollständig auf Umfang abgeschweißt, so dass hier noch eine Eingangsöffnung zum Einfüllen der Silikonmischung vorhanden ist. Über die Öffnung wird zunächst die erste Kammer befüllt, die in Fig. 1 mit 14 bezeichnet ist. Zur Bildung der ersten Kammer wird eine Formhälfte 12 mit einer tiefen Höhlung in die Form eingelegt. Die in der ersten Kammer enthaltene Silikonschicht wird entweder bei Raumtemperatur oder in einem kurzen Ofengang, beispielsweise bei 100 °C in wenigen Minuten vernetzt. Dabei ist das in der ersten Kammer verwendete Silikon so formuliert, dass es bei Raumtemperatur innerhalb weniger Minuten zumindest so stark vernetzt, dass bei den weiteren Fertigungsschritten die gewünschte Schichtdicke und - verteilung erhalten bleibt. Die Höhlung der Formhälfte 12 ist so gestaltet, dass nur ein Hohlraum in diesem Werkzeug für die dünne Schicht freibleibt. Das Silikon in der ersten Kammer ist ein Silikonkautschuk, der ohne Spaltprodukte im Additionsverfahren vernetzt. Die Weichheit wird in Penetrationseinheiten gemessen und bewegt sich im Normalfall von 170 bis 230 Einheiten.

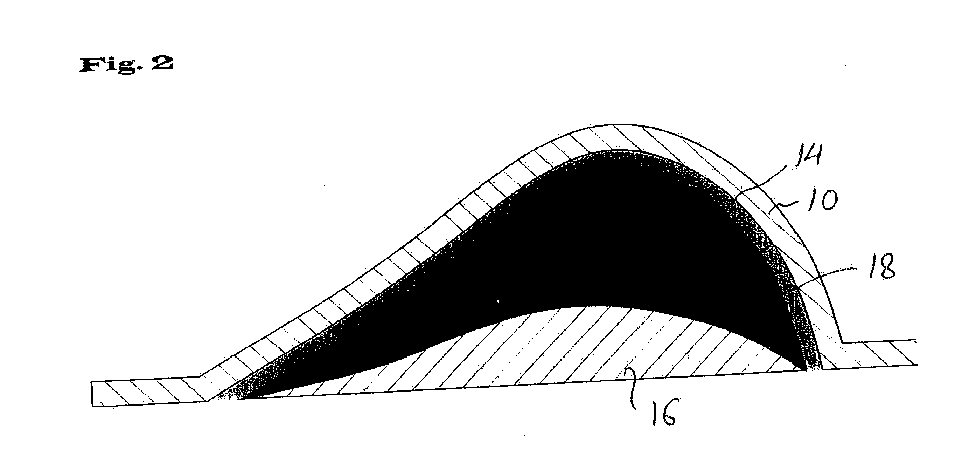

Nach Bildung einer äußeren ersten Silikonschicht, die vorzugsweise eine Dicke zwischen 2 und 10 mm aufweist, wird die zweite Kammer, die die Hauptkammer darstellt, befüllt. Hier wird ein Leichtsilikon, also eine Mischung von Silikon und Hohlkugelfüllstoff, eingefüllt. Anschließend wird eine neue Formhälfte, wie in Fig. 2 dargestellt, nämlich eine flache Formhälfte 16 aufgesetzt. Diese Formhälfte ist so gestaltet, dass die zweite Kammer mit dem Leichtsilikon umschlossen ist. Diese zweite Kammer ist in der Fig. 2 mit 18 bezeichnet. Die entsprechend Fig. 2 vorbereitet Form durchläuft anschließend den Hauptofenprozeß. Die Temperatur im Ofenprozeß richtet sich dabei vornehmlich nach der Erweichungstemperatur und den Erweichungszeiten der den Beutel bildenden Polyurethanfolie. Die Temperatur ist so eingestellt, dass bei der Polyurethanfolie eine dauerhafte, plastische Verformung erreicht wird, ohne dass die Folie jedoch thermisch beschädigt wird. Üblicherweise werden hier Folientemperaturen von ca. 130 °C eingestellt.

Nach diesem Fertigungsschritt wird die Prothese aus der Form genommen und die überstehende Folie beispielsweise mit einem scharfen Messer, einer Schere oder einem Stanzwerkzeugs abgetrennt.

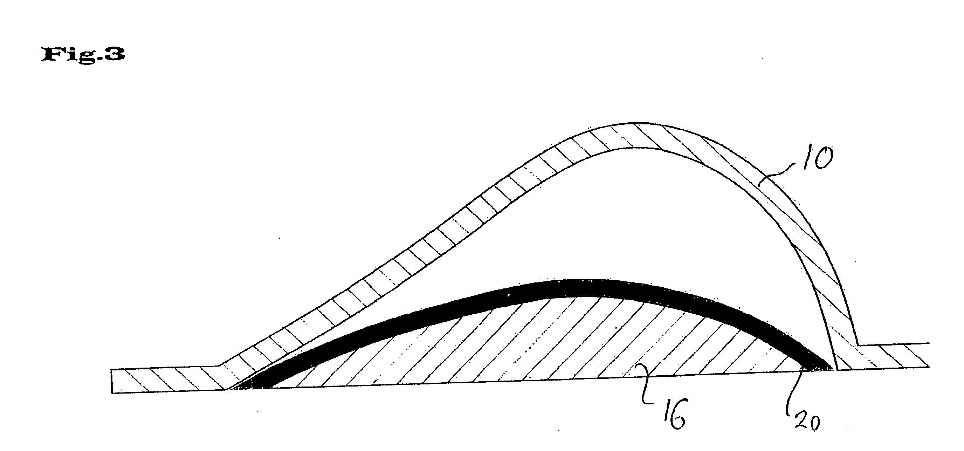

In der Fig. 3 ist eine alternative Verfahrensweise gezeigt. Hier ist eine membranähnliche Höhlung 20 vorhanden, die in ihrer Form einer Folienlage gleicht, die durch Vakuum oder Luftdruck verformt wurde. Das hier verwendete Verfahren entspricht im wesentlichen dem zuvor beschriebenen Verfahren. Bei dem zuvor beschriebenen Verfahren erweist es sich als problematisch, das Vakuum mit hoher Präzision zu halten. Dies wird noch erschwert, wenn die Folienlagen durch Erwärmung weicher werden. Die Verwendung der membranähnlichen Höhlung ermöglicht es, die Kontur in einem definierten Zustand zu halten.

Gegenüber dem Herstellverfahren unter Verwendung einer tiefen Höhlung weist das vorliegende Verfahrenden Vorteil auf, dass das Füllvolumen variiert werden kann. Die membranähnliche Höhlung muß darüber hinaus auch nicht so präzise ausgeführt werden wie bei einer tiefen Höhlung. Allerdings muß die Form waagerecht positioniert werden, bis die erste Schicht ausrechend ausvulkanisiert ist.

Mit diesem Verfahren ist es auch möglich eine dritte Kammer, auf der Körper zugewandten Seite, zu fertigen. Dabei kann die dritte Kammer zeitnah mit der ersten, äußeren Kammer gefüllt werden und zusammen, zumindest teilweise, ausvulkanisiert werden oder in zwei separaten Füll- und Vulkanisationsschritten gefertigt werden. Anschließend wird die mittlere Kammer mit Leichtsilikon gefüllt und vollständig ausvulkanisiert.

Die dritte Kammer kann aber auch als extrem weiche Schicht ausgebildet werden, wie in EP 0 320 590 (Mulligan) beschrieben. Dabei wird entweder die mittlere, mit Leichtsilikon, und rückseitige Kammer, mit extrem weichen Silikon, zeitnah gefüllt und gemeinsam ausvulkanisiert oder in separaten Schritten gefüllt und ausvulkanisiert.

## Patentansprüche

1. Verfahren zur Herstellung einer Brustprothese, die im wesentlichen aus in Kunststofffolien eingeschweißten, der Brustform nachgebildeten Körpern, vorzugsweise aus einer transparenten additionsvernetzenden Zweikomponenten-Silikonkautschuk-Masse und einer additionsvernetzenden Zweikomponenten-Silikonkautschuk-Masse mit einem beigemischten, weitgehend lichtundurchlässigen, Hohlkugelfüllstoff besteht,
mit folgenden Schritten:
- Schweißen eines Folienbeutels aus mindestens drei Folienlagen zur Erzeugung von mindestens zwei Kammern,
- Einlegen des Folienbeutels in das Prothesenformwerkzeug - Befüllen der außen liegenden Kammer mit einer vorzugsweise bereits bei Umgebungstemperatur oder bei leicht erhöhter Temperatur vernet- zenden, transparenten Masse,
- - Teilweises oder vollständiges Aushärten der transparente Masse, um eine ausreichende Formstabilität für die weiteren Fertigungsschritte zu erreichen
- Befüllen der inneren Kammer mit einer zweiten, weitgehend lichtundurchlässigen Masse, gefüllt mit Leichtfüllstoffen
- Einsetzen einer Formhälfte mit einer flachen, die Endkontur definierende Höhlung, um bei erhöhter Temperatur die in den Kammern befindliche Massen vollständig zu vernetzen.
- Die optionale dritte Kammer kann entweder zusammen mit der äußeren Schicht gefüllt werden, in einer Schichtdicke von vorzugsweise 2-10 mm, in der gleichen Funktion wie die äußere Schicht oder
sie wird zusammen der inneren Kammer gefüllt, in einer Weichheit und Menge wie in dem Amoena Patent EP 0 320 590 (Mulligan) beschrieben.

2. Verfahren nach Anspruch 1, **dadurch gekennzeichnet, dass** zur Bildung der dünnen äußeren Schicht in der äußeren Kammer des Folienbeutels eine Formhälfte mit einer tiefen Höhlung auf die erste Formhälfte aufgesetzt wird, so dass durch den Spalt zwischen den Formhälften die Schichtdicke der äußeren Schicht festgelegt wird.

3. Verfahren nach Anspruch 1, **dadurch gekennzeichnet, dass** zur Bildung der dünnen äußeren Schicht in der äußeren Kammer des Folienbeutels eine Formhälfte mit membranähnlich geformenter Höhlung auf die erste Formhälfte aufgesetzt wird, so dass durch den Spalt zwischen den Formhälften die Schichtdicke der äußeren Schicht festgelegt wird.

4. Verfahren nach Anspruch 1, **dadurch gekennzeichnet, dass** zur Bildung der dünnen äußeren Schicht in der äußeren Kammer Druck und/oder Vakuum auf den in der Form eingespannten aus elastischem Material bestehenden Folienbeutel ausgeübt wird, so dass sich die Kontur der gewünschten Höhlung ergibt.

5. Verfahren nach einem der vorangehenden Ansprüche, **dadurch gekennzeichnet, dass** die den Folienbeutel bildenden Folienlagen aus Polyurethan bestehen.

6. Verfahren nach Anspruch 5, **dadurch gekennzeichnet, dass** die Polyurethanfolien ein Dicke von 40 µm - 100 µm aufweisen.

7. Verfahren nach einem der Ansprüche 1 bis 6, **dadurch gekennzeichnet, dass** die Polyurethanfolien mittels Thermoschweißen verschweißt werden.

8. Verfahren nach einem der Ansprüche 1 bis 7, **dadurch gekennzeichnet, dass** die Polyurethanfolien mittels HF-Schweißen verschweißt werden.

9. Verfahren nach einem der Ansprüche 1 bis 8, **dadurch gekennzeichnet, dass** die Polyurethanfolien mittels Laserschweißen verschweißt werden.

10. Verfahren nach einem der Ansprüche 1 bis 9, **dadurch gekennzeichnet, dass** die in der ersten Kammer des Folienbeutels eingefüllte erste Masse aus einer Silikonmischung besteht, die schon bei Raumtemperatur hinreichend stark vernetzt, um nach wenigen Minuten Reaktionszeit weitgehend formstabil zu sein.

11. Verfahren nach einem der Ansprüche 1 bis 10, **dadurch gekennzeichnet, daß** der in der ersten Kammer des Folienbeutels eingefüllten Masse Farbpigmente beigemengt sind.

12. Verfahren nach einem der Ansprüche 1 bis 11, **dadurch gekennzeichnet, dass** die Weichheit des in der ersten Kammer des Folienbeutels eingefüllten Silikons nach dem Vernetzen in Penetrationseinheiten gemessen zwischen 170 und 230 Einheiten beträgt.

13. Verfahren nach einem der Ansprüche 1 bis 12, **dadurch gekennzeichnet, daß** zur vollständigen Vernetzung der in der zweiten Kammer des Folienbeutels eingefüllten Masse die Temperatur so gewählt wird, dass das Folienmaterial dauerhaft plastisch verformt wird, ohne dabei die Folie dauerhaft zu beschädigen.

14. Verfahren nach Anspruch 13, **dadurch gekennzeichnet, dass** bei Verwendung von Polyurethan als Folienmaterial die Temperatur auf ca. 130° C eingestellt wird.

15. Verfahren nach einem der Ansprüche 1 bis 14, **dadurch gekennzeichnet, dass** der Folienbeutel aus vier Folienlagen unter Bildung von drei Kammern gefertigt wird, wobei die vergleichsweise dünne dritte Kammer auf der Rückseite der Brustprothese angeordnet ist.

16. Verfahren nach Anspruch 15, **dadurch gekennzeichnet, dass** die dritte Kammer zusammen mit der ersten außen liegenden Kammer gefüllt wird.

17. Brustprothese nach einem der Ansprüche 1 bis 16, **dadurch gekennzeichnet, dass** die erste und/oder dritte Kammer jeweils eine Dicke von 2 bis 10 mm aufweisen.

18. Brustprothese nach einem der Ansprüche 1 bis 17, **dadurch gekennzeichnet, dass** die dritte Kammer eine Dicke von 5 bis 50 mm aufweist.
